# EUROPEAN PATENT SPECIFICATION

(11) **EP 4 477 200 B1**
(45) Date of publication and mention of the grant of the patent: **29.07.2026**
(21) Application number: 24166991.0
(22) Date of filing: 27.03.2024
(51) Int. Cl.: A61F 15/00, G07F 11/00

(54) **A TIME DELAY PRODUCT DISPENSER E.G. FOR MENSTRUAL PADS**
ABGABEVORRICHTUNG FÜR ZEITVERZÖGERTE PRODUKTE, Z.B. FÜR MENSTRUATIONSKISSEN
DISTRIBUTEUR DE PRODUIT À RETARDEMENT, PAR EXEMPLE POUR SERVIETTES MENSTRUELLES

(30) Priority: 16.06.2023 SE 2350739
(43) Date of publication of application: 18.12.2024
(73) Proprietor: Kvins Sweden AB, 711 34 Lindesberg (SE)
(72) Inventor: Persson, Håkan, 711 31 Lindesberg (SE)
(74) Representative: Ström & Gulliksson AB

(56) References cited:
- WO-A1-00/10433
- US-A1- 2011 000 760

## Description

### Technical field

The present disclosure generally pertains to dispensing of products. More precisely, the present disclosure relates to a wall-mounted apparatus for time-delayed dispensing of e.g. hygiene products such as tampons and/or menstrual pads.

### Background art

There exist several environments where it may be desirable to offer various items to the public free of charge, but in limited quantities. The items are to be readily accessible to a user, but the supply is to be limited such that a malicious user may not overexploit the service and quickly deplete the supply of items. For example, in public restrooms it may be desirable to offer hygiene products.

The patent application publication WO 2022/060276 A1 discloses a time delay product dispenser for tampons.

The patent application publication US 2011/000760 A1 discloses a coin operable vending apparatus comprising one or two product receptacles for containing products, a respective rotatable discharge chamber positioned below each product receptacle, and a respective coin mechanism for each discharge chamber.

The patent application publication WO 00/10433 A1 discloses a package dispenser for controlled delivery of packages. The package dispenser comprises a single container configured to hold one or two stacks of packages. The container may comprise a series of flexible slots configured to hold a respective package within the container.

### Summary of the invention

The present disclosure aims at providing a low-cost and reliable time delay product dispenser. The time delay product dispenser should allow products of various shapes and sizes to be dispensed, should be easy to refill, and should have a time delay function that is relatively easily to adjust and repair.

The present disclosure provides a time delay product dispenser that comprises a set of containers each adapted for containing a product, wherein the set of containers are interconnected to form a chain structure, a feeding mechanism that is configured to move the set of containers such that the products are successively accessible to a user, an actuator assembly for actuating the feeding mechanism, and a movement damping device to slow down a movement of the actuator assembly.

Advantages of the present disclosure involve that the set or series of containers may be dimensioned to contain products of various shapes and sizes, such as a tampon or a menstrual pad. The movement damping device may be a linear damper, which may be a cost effective movement damping device as compared to e.g. electric movement damping devices or rotary movement damping devices. A linear damper, or linear movement damping device, may also be relatively easy to adjust, repair or replace.

Further features and advantages of the dispenser are disclosed in the appended claims and drawings, and in the following description.

### Brief description of the drawings

Examples are described in more detail below with reference to the appended drawings, in which:
- figure 1: is an isometric view illustrating the interior of the present time delay product dispenser.
- figure 2: corresponds to figure 1 but with a front housing attached to the product dispenser,
- figure 3: is a plan view of the time delay product dispenser of figure **1****,**
- figure 4: is a detailed isometric view of a set of containers, an actuator assembly and a feeding mechanism of the present product dispenser,
- figure 5: is a detailed side view of the actuator assembly and the feeding mechanism of the present product dispenser, and
- figure 6: shows the actuator assembly, the feeding mechanism and a movement damping device of the present product dispenser.

### Detailed description of embodiments

The time delay product dispenser according to the present disclosure will now be described more fully hereinafter. The time delay product dispenser according to the present disclosure may however be embodied in many different forms and should not be construed as limited to the embodiments set forth herein. Rather, these embodiments are provided by way of example so that this disclosure will be thorough and complete, and will fully convey the scope of the present disclosure to those persons skilled in the art.

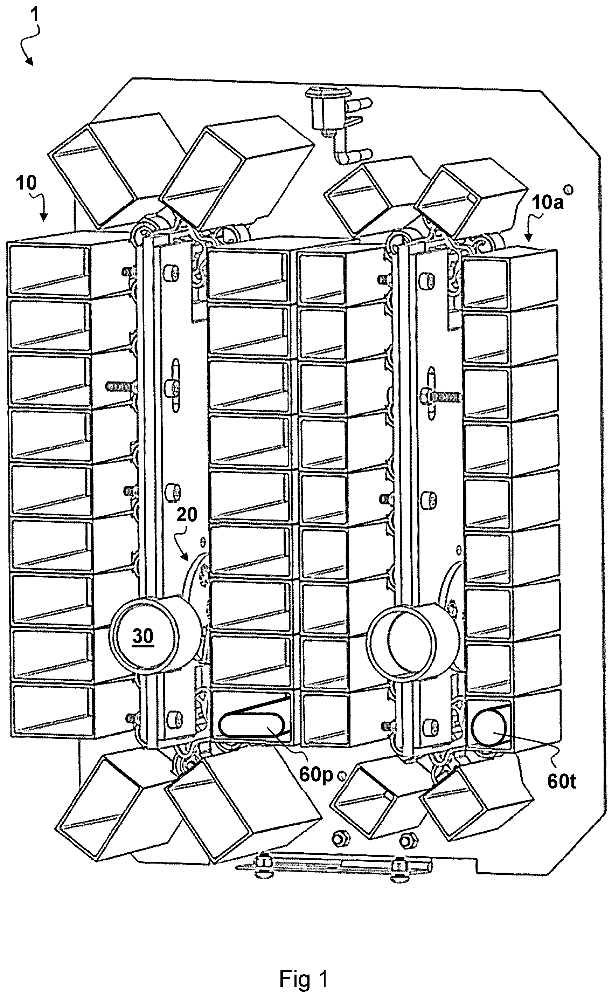

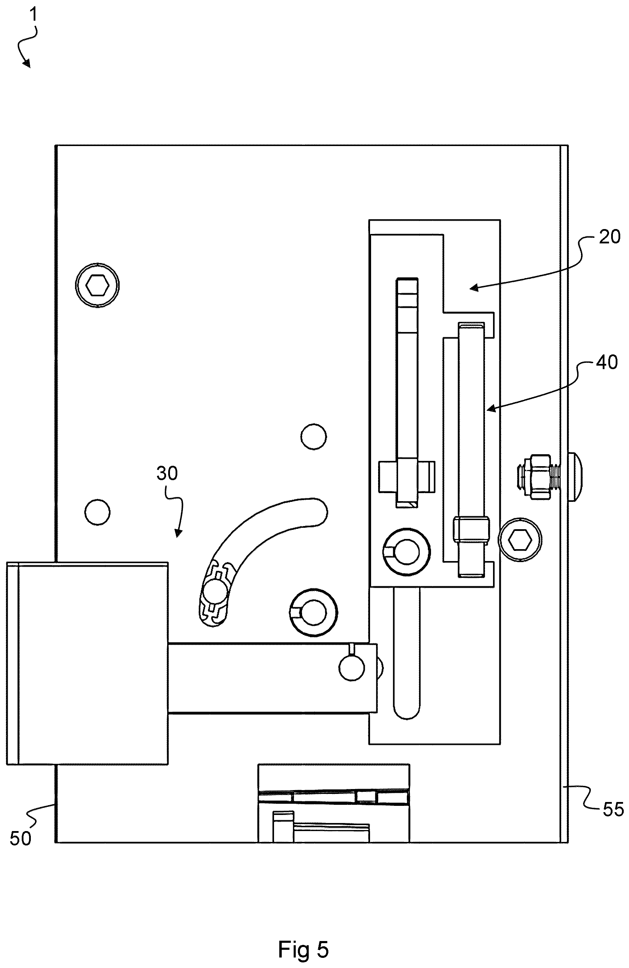

Figures 1 to 6 illustrate a time delay product dispenser 1. The time delay product dispenser 1 or dispenser 1 comprises a set of containers 10 each adapted for containing a product 60. The set of containers 10 are a part of the dispenser 1.

The present dispenser 1 comprises a feeding mechanism 20 that is configured to move the set of containers 10 such that the products contained in the set of containers 10 are successively accessible to a user. The present dispenser 1 further comprises an actuator assembly 30 for actuating or manoeuvring the feeding mechanism 20. As is illustrated, the actuator assembly 30 may drive the feeding mechanism 20. The actuator assembly 30 may be actuated or operated by a user.

The present dispenser 1 comprises a movement damping device 40 to slow down a movement of the actuator assembly 30. Since the movement damping device 40 slows down a movement of the actuator assembly 30, there will be a delay, for example 10 seconds, 20 seconds, or even one or a couple of minutes, before the actuator assembly 30 may again be operated by a user, and thus the present dispenser 1 is referred to as a time delay product dispenser 1.

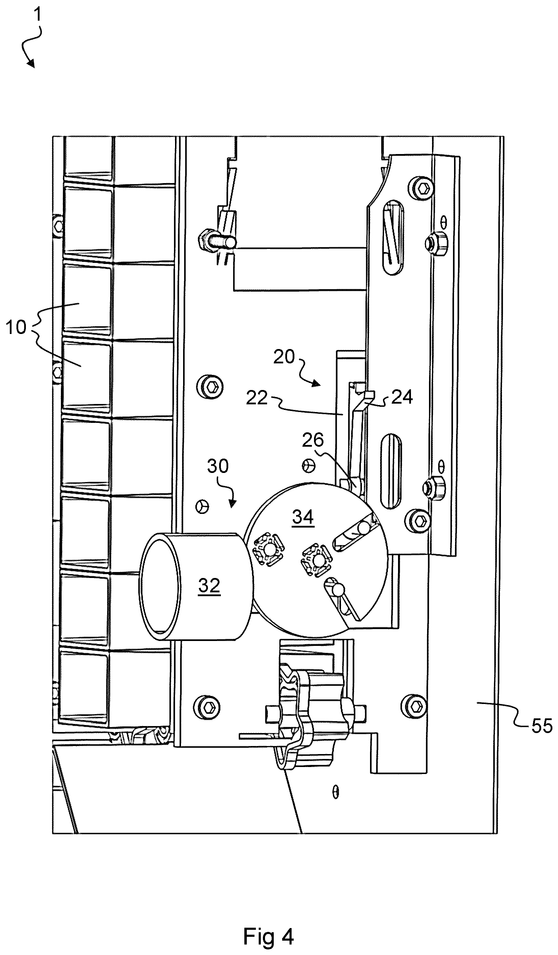

The movement damping device 40 may slow down a return movement (to the left in figures 4 to 6) of the actuator assembly 30, to provide the time delay function. In addition the movement damping device 40 may slow down an opposite actuation movement (to the right in figures 4 to 6) of the actuator assembly 30, to provide a controlled movement of the set of containers 10. The controlled movement may involve hindering the set of containers 10 from moving too fast, which may cause an unpleasant noise and may dislocate a product 60 contained in the set of containers 10. A dampened actuation movement may reduce wear and provide an improved user experience.

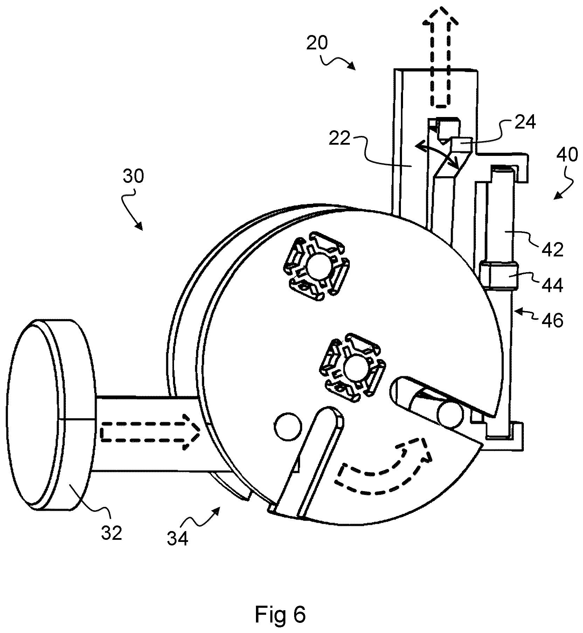

Referring in particular to figure 6, the present movement damping device 40 is a linear damper. The present linear damper 40 comprises a movable part 42 that is operatively connected to the actuator assembly 30 and a stationary part 44 that is attached to a front housing 50 of the time delay product dispenser 1. In some detail, the movable part 42 of the linear damper 40 is in the present example attached to the feeding mechanism 20 which in turn in connected to the actuator assembly 30. The stationary part 44 may be attached, e.g. screwed, to a rear housing portion 55 or a chassis of the dispenser 1. The present linear damper 40 is configured such that a relative movement between the movable part 42 and the stationary part 44 is counteracted by a resistive force between the movable part 42 and the stationary part 44. The present linear damper 40 dampens a movement of the feeding mechanism 20 which in turn dampens a movement of the actuator assembly 30.

The resistive force may be a friction force. A resistive friction force may be generated by the movable part 42 and the stationary part 44 being configured for mutual tight engagement. **In** some embodiments, the movable part 42 and the stationary part 44 may comprise cooperating friction surfaces of different materials. The movable part 42 may be made of polymer material or may comprise a friction surface of polymer material, and the stationary part 44 may be made of metal or comprise a friction surface of metal, or vice versa.

Alternatively, the resistive force may be a viscous force. A resistive viscous force may be generated by a viscous liquid 46 being arranged between the movable part 42 and the stationary part 44. As is to be apprehended, in the latter examples the movable part 42 and the stationary part 44 may be configured for relatively mutual tight engagement. The viscous liquid 46 may be a grease or a viscous oil. Examples of suitable viscous liquids 46 are the damping greases PG-44A and 774VH.

**In** the present example, as is best illustrated in figure 6, the movable part 42 and the stationary part 44 slidingly engage one another. **In** some detail, the movable part 42 is elongated and the stationary part 44 comprises a through hole that receives the elongated movable part 42. As is illustrated, the movable part 42 may be a rod of circular cross-section and the stationary part 44 may comprise a circular through hole. **In** the present example, the stationary part 44 is annular, more precisely ring-shaped. The length of the elongated movable part 42 may essentially correspond to the distance between two neighbouring containers 10 of the set of containers 10.

Referring to the figures, the actuator assembly 30 may comprise a linearly movable activation element 32 to be operated by a user. In the present example, the activation element 32 is a push-button. The linearly movable activation element 32 may be arranged such that an actuation movement is directed through the front housing 50 of the dispenser 1.

The present linearly movable activation element 32 is arranged on the front of the dispenser 1. In other words, the linearly movable activation element 32 may be operated by the user pressing the linearly movable activation element 32 on a front side of the dispenser 1. Such a solution generally improved usability, and limits the structural requirements as regards the strength of the dispenser 1 and its suspension on e.g. a wall.

In the present example, as is best shown in figures 4 and 6, the actuator assembly 30 comprises a translating mechanism 34 that is adapted to translate a linear movement of the linearly movable activation element 32 into an at least party vertical linear movement of the feeding mechanism 20. The dashed arrows in figure 6 illustrate how the push button 32 may be pushed in a horizontal direction by a user, how that horizontal movement results in a rotary movement of the translating mechanism 34, and how that rotary movement results in a vertical movement of the feeding mechanism 20.

In the present example, the feeding mechanism 20 comprises a main body 22. The main body 22 may be made of metal and is of a sufficient volume such that a vertical movement of the feeding mechanism 20 results in a stored potential energy. In other words, the feeding mechanism 20 may comprises or be connected to a weight that may be lifted to store energy. The stored energy may be converted into kinetic energy to reverse the movement of the feeding mechanism 20, the translating mechanism 34 and the push button 32. In an undisclosed embodiment, the dispenser 1 may instead comprise a spring that may be charged by the user moving the actuator assembly 30.

The weight of the feeding mechanism 20, or the energy of the spring, may be used to by gravity return the actuator assembly 30 (here, the push button 32) to its starting position.

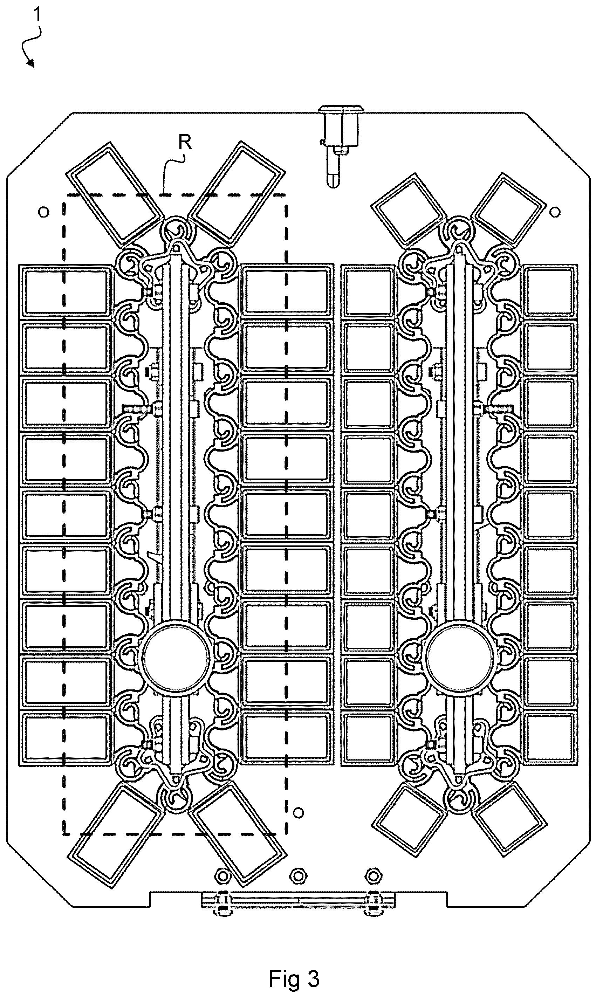

In the present example, the series or set of containers 10 are interconnected to form a chain structure. Referring in particular to figures 1 and 3, the present set of containers 10 may best be referred to as a carousel of containers 10. The present feedings mechanism 20 is configured to engage the set of containers 10 to move, or forward, the chain of containers 10 when the activation element 32 of the actuator assembly is operated by a user. As is illustrated in figure 6, the feeding mechanism 20 may comprise an engagement protrusion 24 that is adapted to engage the set of containers 10. More precisely, the main body 22 may comprise an engagement protrusion in the form of a hook 24 that may drive the chain of containers 10. When the user pushes the push button 32, the hook 24 is moved (here upwards) a distance that corresponds to the distance between the storage compartments of two adjacent containers 10. The hook 24 may engage one of the containers, or an undulating chain structure (figure 3) that may connect the container to one another in series, to move the set of containers 10 a distance that corresponds to the stroke of the actuator assembly 30.

The protrusion 24 may be movable between an engagement position, in which the set of containers 10 is engaged, and a release position, in which the set of containers 10 is not engaged. For example, the protrusion 24 may be movably attached to the main body 22. In the present example, the feeding mechanism comprises a hinge 26 that movably attaches the protrusion 24 to the main body 22, see e.g. figure 4. The double-headed arrow in figure 6 illustrates that the protrusion 24 is movable towards and away from the viewer.

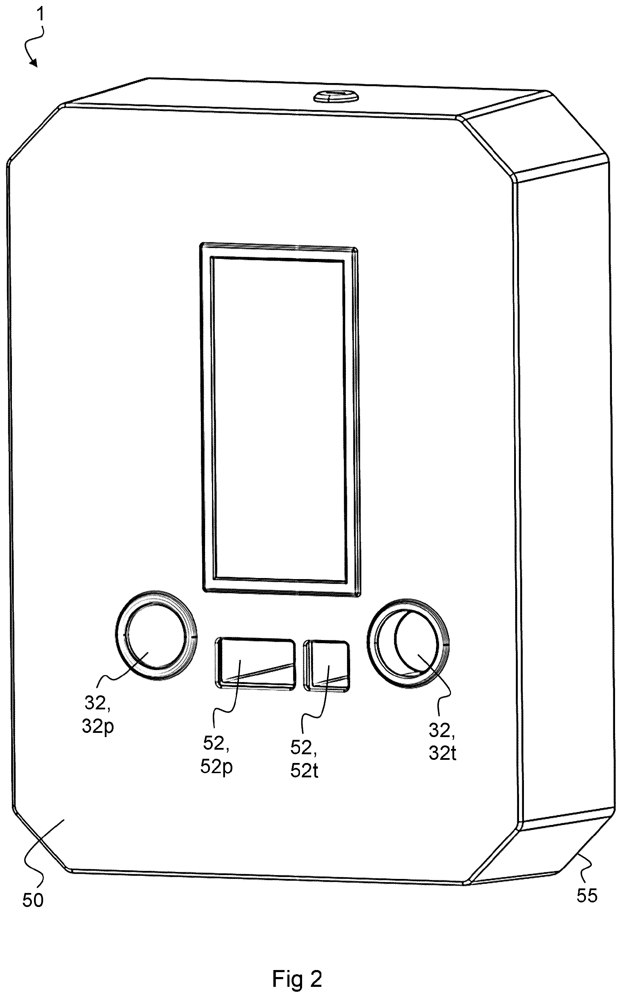

Referring to figures 1 and 2, the front housing 50 of the dispenser 1 may comprise an access opening 52. In the present example, the access opening 52 is arranged on the front of the dispenser 1. Thus, both the linearly movable activation element 32 and the access opening 52 may be arranged on the front of the dispenser 1, i.e. opposite to the wall on which the dispenser is mounted. As is apprehended from figures 1 and 2, the present dispenser 1 is configured such that a product 60 contained in one container 10 of the set of containers is accessible to a user through the access opening 52 when said container 10 is aligned with said access opening 52. Thus, the user may reach through the access opening 52 and into the container 10 to pick the product 60.

As is illustrated, the access opening 52 may be of a shape that essentially corresponds to the cross-section of the containers 10 of the set of containers.

Each one of the set of containers 10 may be sized to hold a product 60 in the form of a menstrual pad 60p (figure 1). For example, each container may be essentially box-shaped. Typically, referring to figure 1, one of the container of the set of containers 10 may be tubular and elongated. Each container may have a width of 20 millimetres to 100 millimetres. The depth may be 40 to 200 millimetres. The height may be 20 to 60 millimetres. Typically, referring again to figure 1, each container for a menstrual pad 60p may be tubular and have a width and a depth that exceed the height. In the present example, each menstrual pad container has a width of 50 millimetres, a depth of 90 millimetres and a height of 25 millimetres, particularly suitable ranges for the width, depth and height being 40-60 x 70-110 x 20-30 millimetres.

Referring to figures 1 to 3, the dispenser 1 may comprise a first and a second set of containers 10, 10a. Both sets of containers 10, 10a may comprise an individual time delay functionality as described above. Thus, the dispenser 1 may be adapted to dispense two different products 60p, 60t independently. There may be a fist and a second feeding mechanism 20, 20a, a first and a second actuator assembly 30, 30a, a first and a second movement damping device 40, 40a. The present dispenser 1 thus comprises a first activation element 32p and an associated first access opening 52p, and a second activation element 32t and an associated second access opening 52t.

In the present example, the each one of the first set of containers 10 is adapted to contain a menstrual pad 60p and each one of the second set of containers 10a is adapted to contain a tampon 60t. Thus, for example, during a restroom visit a user may obtain one menstrual pad 60p (pad push-button 32p and pad access opening 52p) and one tampon 60t (tampon push-button 32t and tampon access opening 52t). The time delay product dispenser 1 may be set such that no further menstrual pad 60p or tampon 60t is accessible before a predetermined time has lapsed.

The time, or time delay, may be set by selecting or adjusting the movement damping device. For example, the linear damper may be adjusted to provide a suitable resistive friction force or resistive viscous force. The friction force may be adjustable by adjusting a nondisclosed tensioning screw that adjusts a clamping force between the movable part 42 and the stationary part 44 of the linear damper 40. Similarly, the viscous force may be adjustable by nondisclosed tensioning screw, or by selecting an appropriate viscous liquid 46 or the amount of viscous liquid 46.

The chain structure may, as in the illustrated example, generally have the shape of a rectangle R (figure 3). Such a shape may be advantageous for providing a large amount of products 60 within a relatively compact dispenser 1.

Examples of other products that may be contained in one or more sets pf container of the dispenser 1 are solid soap, with or without an enclosing packaging, or portion packs of liquid soap, shampoo and conditioner. Referring to the above, the present dispenser 1 may be manually or mechanically operated, i.e. propeller by the user actuating the actuator assembly 30. No connection to mains, or internal battery, is required. The time delay feature prevents a malicious person from obtaining a large number of products within a short period of time.

Modifications and other variants of the described embodiments will come to mind to one skilled in the art having benefit of the teachings presented in the foregoing description and associated drawings. Therefore, it is to be understood that the embodiments are not limited to the specific example embodiments described in this disclosure and that modifications and other variants are intended to be included within the scope of this disclosure.

Furthermore, although specific terms may be employed herein, they are used in a generic and descriptive sense only and not for purposes of limitation. Therefore, a person skilled in the art would recognize numerous variations to the described embodiments that would still fall within the scope of the appended claims. As used herein, the terms "comprise/comprises" or "include/includes" do not exclude the presence of other elements or steps. Furthermore, although individual features may be included in different claims (or embodiments), these may possibly advantageously be combined, and the inclusion of different claims (or embodiments) does not imply that a certain combination of features is not feasible and/or advantageous. In addition, singular references do not exclude a plurality. Finally, reference signs in the claims are provided merely as a clarifying example and should not be construed as limiting the scope of the claims in any way.

## Claims

1. A time delay product dispenser (1) comprising
- a set of containers (10) each adapted for containing a product (60), wherein the set of containers (10) are interconnected to form a chain structure,
- a feeding mechanism (20) that is configured to move the set of containers (10) such that the products are successively accessible to a user,
- an actuator assembly (30) for actuating the feeding mechanism (20), and
- a movement damping device (40) to slow down a movement of the actuator assembly (30).

2. The time delay product dispenser (1) of claim 1, wherein the movement damping device (40) is a linear damper.

3. The time delay product dispenser (1) of claim 2, wherein the linear damper (40) comprises a movable part (42) that is operatively connected to the actuator assembly (30) and a stationary part (44) that is attached to a housing (50) of the time delay product dispenser (1), and wherein the linear damper (40) is configured such that a relative movement between the movable part (42) and the stationary part (44) is counteracted by a resistive force between the movable part (42) and the stationary part (44).

4. The time delay product dispenser (1) of claim 3, wherein the movable part (42) and the stationary part (44) are configured for mutual tight engagement and the resistive force is a friction force, or wherein the linear damper (40) comprises a viscous liquid (46) that is arranged between the movable part (42) and the stationary part (44) and the resistive force is a viscous force.

5. The time delay product disperser (1) of claim 3 or 4, wherein the movable part (42) and the stationary part (44) slidingly engage one another.

6. The time delay product dispenser (1) according to any of claims 3 to 5, wherein the actuator assembly (30) comprise a linearly movable activation element (32) to be operated by a user.

7. The time delay product dispenser (1) of claim 6, wherein the actuator assembly (30) comprises a translating mechanism (34) that is adapted to translate a linear movement of the linearly movable activation element (32) into an at least party vertical linear movement of the feeding mechanism (20).

8. The time delay product dispenser (1) of any preceding claim, comprising a weight or a spring and configured such that the actuator assembly (30), after having been actuated, returns to a starting position by a spring-force of the spring or by gravity of the weight, and wherein the movement damping device (40) is adapted to dampen the movement of the actuator assembly (30 to its starting position.

9. The time delay product dispenser (1) of any preceding claim, comprising a housing (50) with an access opening (52), wherein the time delay product dispenser (1) is configured such that a product (60) contained in a container (10) is accessible to a user through the access opening (52) when said container (10) is aligned with said access opening (52).

10. The time delay product dispenser (1) of any preceding claim, wherein the each one of the set of containers (10) is sized to hold a product (60) in the form of a menstrual pad (60p).

11. The time delay product dispenser (1) of any preceding claim, comprising a first and a second set of containers (10, 10a), feeding mechanisms (20, 20a) actuator assemblies (30, 30a) and movement damping devices (40, 40a), such that the time delay product dispenser (1) is adapted to dispense two different products (60p, 60t) independently.

12. The time delay product dispenser (1) of claim 11, wherein each one of the first set of containers (10) is adapted to contain a menstrual pad (60p) and each one of the second set of containers (10a) is adapted to contain a tampon (60t).

## Patentansprüche

1. Zeitverzögerte Produktabgabevorrichtung (1), umfassend:
einen Satz von Behältern (10), von denen jeder dazu ausgebildet ist, ein Produkt (60) zu enthalten, wobei der Satz von Behältern (10) miteinander verbunden ist, um eine Kettenstruktur zu bilden,
einen Zuführmechanismus (20), der dazu eingerichtet ist, den Satz von Behältern (10) so zu bewegen, dass die Produkte für einen Benutzer nacheinander zugänglich sind,
eine Betätigungsanordnung (30) zum Betätigen des Zuführmechanismus (20), und
eine Bewegungsdämpfungsvorrichtung (40), um eine Bewegung der Betätigungsanordnung (30) zu verlangsamen.

2. Zeitverzögerte Produktabgabevorrichtung (1) nach Anspruch 1, wobei die Bewegungsdämpfungsvorrichtung (40) ein Lineardämpfer ist.

3. Zeitverzögerte Produktabgabevorrichtung (1) nach Anspruch 2, wobei der Lineardämpfer (40) ein bewegliches Teil (42), das mit der Betätigungsanordnung (30) wirkverbunden ist, und ein stationäres Teil (44) umfasst, das an einem Gehäuse (50) der zeitverzögerten Produktabgabevorrichtung (1) befestigt ist, und wobei der Lineardämpfer (40) so eingerichtet ist, dass einer relativen Bewegung zwischen dem beweglichen Teil (42) und dem stationären Teil (44) durch eine Widerstandskraft zwischen dem beweglichen Teil (42) und dem stationären Teil (44) entgegengewirkt wird.

4. Zeitverzögerte Produktabgabevorrichtung (1) nach Anspruch 3, wobei das bewegliche Teil (42) und das stationäre Teil (44) für einen gegenseitigen engen Eingriff eingerichtet sind und die Widerstandskraft eine Reibungskraft ist, oder wobei der Lineardämpfer (40) eine viskose Flüssigkeit (46) umfasst, die zwischen dem beweglichen Teil (42) und dem stationären Teil (44) angeordnet ist, und die Widerstandskraft eine viskose Kraft ist.

5. Zeitverzögerte Produktabgabevorrichtung (1) nach Anspruch 3 oder 4, wobei das bewegliche Teil (42) und das stationäre Teil (44) gleitend ineinandergreifen.

6. Zeitverzögerte Produktabgabevorrichtung (1) nach einem der Ansprüche 3 bis 5, wobei die Betätigungsanordnung (30) ein linear bewegliches Aktivierungselement (32) umfasst, das von einem Benutzer zu betätigen ist.

7. Zeitverzögerte Produktabgabevorrichtung (1) nach Anspruch 6, wobei die Betätigungsanordnung (30) eine Umsetzungseinrichtung (34) umfasst, die dazu ausgebildet ist, eine lineare Bewegung des linear beweglichen Aktivierungselements (32) in eine mindestens teilweise vertikale lineare Bewegung des Zuführmechanismus (20) umzusetzen.

8. Zeitverzögerte Produktabgabevorrichtung (1) nach einem der vorhergehenden Ansprüche, die ein Gewicht oder eine Feder umfasst und so eingerichtet ist, dass die Betätigungsanordnung (30), nachdem sie betätigt wurde, durch eine Federkraft der Feder oder durch die Schwerkraft des Gewichts in eine Ausgangsposition zurückkehrt, und wobei die Bewegungsdämpfungsvorrichtung (40) dazu ausgebildet ist, die Bewegung der Betätigungsanordnung (30) in ihre Ausgangsposition zu dämpfen.

9. Zeitverzögerte Produktabgabevorrichtung (1) nach einem der vorhergehenden Ansprüche, umfassend ein Gehäuse (50) mit einer Zugangsöffnung (52), wobei die zeitverzögerte Produktabgabevorrichtung (1) so eingerichtet ist, dass ein in einem Behälter (10) enthaltenes Produkt (60) durch die Zugangsöffnung (52) für einen Benutzer zugänglich ist, wenn der Behälter (10) mit der Zugangsöffnung (52) ausgerichtet ist.

10. Zeitverzögerte Produktabgabevorrichtung (1) nach einem der vorhergehenden Ansprüche, wobei jeder Behälter des Satzes von Behältern (10) so bemessen ist, dass er ein Produkt (60) in Form einer Menstruationsbinde (60p) aufnehmen kann.

11. Zeitverzögerte Produktabgabevorrichtung (1) nach einem der vorhergehenden Ansprüche, umfassend einen ersten und einen zweiten Satz von Behältern (10, 10a), Zuführmechanismen (20, 20a), Betätigungsanordnungen (30, 30a) und Bewegungsdämpfungsvorrichtungen (40, 40a), sodass die zeitverzögerte Produktabgabevorrichtung (1) dazu ausgebildet ist, zwei verschiedene Produkte (60p, 60t) unabhängig voneinander auszugeben.

12. Zeitverzögerte Produktabgabevorrichtung (1) nach Anspruch 11, wobei jeder Behälter des ersten Satzes von Behältern (10) dazu ausgebildet ist, eine Menstruationsbinde (60p) zu enthalten, und jeder Behälter des zweiten Satzes von Behältern (10a) dazu ausgebildet ist, einen Tampon (60t) zu enthalten.

## Revendications

1. Distributeur de produit à temporisation (1) comprenant
- un ensemble de récipients (10) chacun adapté pour contenir un produit (60), lequel ensemble de récipients (10) est interconnecté pour former une structure en chaîne,
- un mécanisme d'alimentation (20) qui est configuré pour déplacer l'ensemble de récipients (10) de sorte que les produits soient successivement accessibles à un utilisateur,
- un ensemble d'actionneur (30) pour actionner le mécanisme d'alimentation (20), et
- un dispositif d'amortissement de mouvement (40) pour ralentir un mouvement de l'ensemble d'actionneur (30).

2. Distributeur de produit à temporisation (1) selon la revendication 1, lequel dispositif d'amortissement de mouvement (40) est un amortisseur linéaire.

3. Distributeur de produit à temporisation (1) selon la revendication 2, lequel amortisseur linéaire (40) comprend une partie mobile (42) qui est reliée fonctionnellement à l'ensemble d'actionneur (30) et une partie fixe (44) qui est fixée à un boîtier (50) du distributeur de produit à temporisation (1), et lequel amortisseur linéaire (40) est configuré de sorte qu'un mouvement relatif entre la partie mobile (42) et la partie fixe (44) est contrecarré par une force de résistance entre la partie mobile (42) et la partie fixe (44).

4. Distributeur de produit à temporisation (1) selon la revendication 3, dans lequel la partie mobile (42) et la partie fixe (44) sont configurées pour un engagement serré mutuel et la force de résistance est une force de frottement, ou dans lequel l'amortisseur linéaire (40) comprend un liquide visqueux (46) qui est disposé entre la partie mobile (42) et la partie fixe (44) et la force de résistance est une force visqueuse.

5. Distributeur de produit à temporisation (1) selon la revendication 3 ou 4, dans lequel la partie mobile (42) et la partie fixe (44) s'engagent par glissement l'une avec l'autre.

6. Distributeur de produit à temporisation (1) selon l'une quelconque des revendications 3 à 5, dans lequel l'ensemble d'actionneur (30) comprend un élément d'activation mobile linéairement (32) à actionner par un utilisateur.

7. Distributeur de produit à temporisation (1) selon la revendication 6, dans lequel l'ensemble d'actionneur (30) comprend un mécanisme de translation (34) qui est adapté pour traduire un mouvement linéaire de l'élément d'activation mobile linéairement (32) en un mouvement linéaire vertical au moins partiel du mécanisme d'alimentation (20).

8. Distributeur de produit à temporisation (1) selon l'une quelconque des revendications précédentes, comprenant un poids ou un ressort et configuré de sorte que l'ensemble d'actionneur (30), après avoir été actionné, revienne à une position de départ par une force de ressort du ressort ou par la gravité du poids, et dans lequel le dispositif d'amortissement de mouvement (40) est adapté pour amortir le mouvement de l'ensemble d'actionneur (30) vers sa position de départ.

9. Distributeur de produit à temporisation (1) selon l'une quelconque des revendications précédentes, comprenant un boîtier (50) avec une ouverture d'accès (52), dans lequel le distributeur de produit à temporisation (1) est configuré de sorte qu'un produit (60) contenu dans un récipient (10) soit accessible à un utilisateur à travers l'ouverture d'accès (52) lorsque ledit récipient (10) est aligné avec ladite ouverture d'accès (52).

10. Distributeur de produit à temporisation (1) selon l'une quelconque des revendications précédentes, dans lequel chacun de l'ensemble de récipients (10) est dimensionné pour contenir un produit (60) sous la forme d'une serviette hygiénique (60p).

11. Distributeur de produit à temporisation (1) selon l'une quelconque des revendications précédentes, comprenant un premier et un second ensemble de récipients (10, 10a), des mécanismes d'alimentation (20, 20a), des ensembles d'actionneur (30, 30a) et des dispositifs d'amortissement de mouvement (40, 40a), de sorte que le distributeur de produit à temporisation (1) soit adapté pour distribuer deux produits différents (60p, 60t) indépendamment.

12. Distributeur de produit à temporisation (1) selon la revendication 11, dans lequel chacun du premier ensemble de récipients (10) est adapté pour contenir une serviette hygiénique (60p) et chacun du second ensemble de récipients (10a) est adapté pour contenir un tampon (60t).
